(19) **Europäisches Patentamt / European Patent Office / Office européen des brevets**

(11) **EP 1 543 833 B1**

(12) **EUROPEAN PATENT SPECIFICATION**

(45) Date of publication and mention
of the grant of the patent:
**06.09.2017 Bulletin 2017/36**

(51) Int Cl.:
*A61K 31/721* *(2006.01)*    *A61P 1/04* *(2006.01)*
*A61P 31/00* *(2006.01)*    *A61P 37/04* *(2006.01)*
*A61P 37/08* *(2006.01)*    *A61P 43/00* *(2006.01)*

(21) Application number: **03765361.5**

(22) Date of filing: **23.07.2003**

(86) International application number:
**PCT/JP2003/009324**

(87) International publication number:
**WO 2004/009099 (29.01.2004 Gazette 2004/05)**

(54) **PHOSPHORYLATED DEXTRAN**

PHOSPHORYLIERTES DEXTRAN

DEXTRAN PHOSPHORYL

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **23.07.2002 JP 2002213305
27.02.2003 JP 2003050739**

(43) Date of publication of application:
**22.06.2005 Bulletin 2005/25**

(73) Proprietor: **Meiji Co., Ltd.
Chuo-ku
Tokyo 104-8306 (JP)**

(72) Inventors:
 • **SAITO, Tadao
 Sendai-shi, Miyagi 981-0943 (JP)**
 • **KITAZAWA, Haruki
 Sendai-shi,
 Miyagi 981-3213 (JP)**

(74) Representative: **Grünecker Patent- und
Rechtsanwälte
PartG mbB
Leopoldstraße 4
80802 München (DE)**

(56) References cited:
 **JP-A- 51 041 083**

 • **DATABASE WPI Week 197621 6 April 1976
 (1976-04-06) Thomson Scientific, London, GB;
 AN 1976-38836X XP002555178 & JP 51 041083 A
 6 April 1976 (1976-04-06)**
 • **M. SUZUKI ET AL: "Preparation and antitumor
 activity of o-palmitoyldextran phosphates,
 o-palmitoyldextrans, and dextran phosphate"
 CARBOHYDRATE RESEARCH, vol. 53, 1977,
 pages 223-229, XP002555179**
 • **M. SUZUKI: "Dextran derivatives in single and
 combination chemotherapy against
 transplantable mouse ascites and solid tumors"
 CANCER RESEARCH, vol. 37, 1977, pages
 3448-3454, XP002555180**
 • **SACCO D ET AL: "A re-investigation of the
 phosphorylation of dextran with polyphosphoric
 acid: Evidence for the formation of different types
 of phosphate moieties" CARBOHYDRATE
 RESEARCH, ELSEVIER SCIENTIFIC
 PUBLISHING COMPANY. AMSTERDAM, NL, vol.
 184, 31 December 1988 (1988-12-31), pages
 193-202, XP026618203 ISSN: 0008-6215 [retrieved
 on 1988-12-31]**
 • **TARELLI E ET AL: "Drying from
 Phosphate-Buffered Solutions Can Result in the
 Phosphorylation of Primary and Secondary
 Alcohol Groups of Saccharides, Hydroxylated
 Amino Acids, Proteins, and Glycoproteins"
 ANALYTICAL BIOCHEMISTRY, ACADEMIC
 PRESS INC, NEW YORK, vol. 222, no. 1, 1 October
 1994 (1994-10-01), pages 196-201, XP024762919
 ISSN: 0003-2697 [retrieved on 1994-10-01]**

**Description**

Technical Field

The present invention relates to pharmaceutical compositions with immunopotentiating activity, comprising a phosphorylated dextran as an active ingredient.

Background Art

Polysaccharides contain many hydroxyl groups. By introducing a certain type of substituent into all or some of these hydroxyl groups, new characteristics, which were not observed before the substitution, can sometimes emerge. For example, carboxymethyl cellulose (CM-cellulose), in which 40% or more of the hydroxyl groups in cellulose are substituted with carboxymethyl groups, is soluble in water and forms a stable high-viscosity colloidal solution. Thus, CM-cellulose is applicable as a stabilizer for processed foods, such as ice cream and jam. For another example, the introduction of carboxymethyl groups into the polysaccharide "Kefiran", which is isolated from Kefir grains comprising seed bacteria for a fermented milk product called "Kefir", resulted in eight-fold or more increase in viscosity, and this increase in viscosity has thus increased application of Kefir as a food.

In this way, polysaccharide derivatives prepared through some sort of chemical modification are attracting attention as foods, and studies aiming at their application in medical fields are also being carried out. Many such studies utilize an enhancement or induction of biological activity by introducing substituents into polysaccharides. For example, dextran sulfates (a molecular weight of about 6,500; a sulfur content of 16-18%), which are prepared by sulfating dextrans, have heparin-like anticoagulant activity and low toxicity, and are thus being clinically applied. These polysaccharides are also highly used as therapeutic agents for hyperlipidemia. Furthermore, dextran sulfates have long been known to show an antiproliferative effect on various viruses, and have also been reported as effective in suppressing the proliferation of HIV (Human Immunodeficiency Virus). Thus, dextran sulfates have recently drawn attention as anti-AIDS (Acquired Immunodeficiency Syndrome) agents. It has been reported that heparin and mannan sulfates show an antiproliferative effect on viruses, but that corresponding nonsulfated dextrans and mannans themselves have no suppressing effect (see non-patent document 1). Therefore, the sulfate group is thought to be a factor in expression of this activity.

In contrast to dextran sulfates, there are few reports describing the biological activity of phosphorylated dextrans, which are phosphorylated dextran derivatives. Suzuki et al. synthesized a phosphorylated dextran by introducing phosphate groups into a dextran (molecular weight of 38,000) via a reaction with polyphosphoric acid in a formamide solution (see non-patent document 2). Although the resultant phosphorylated dextran was reported to suppress the growth of solid tumor Sarcoma-180 (S-180), which was transplanted into the peritoneal cavities of mice, details regarding the expression of immunological activity are still unknown.

Conventional methods for preparing phosphorylated dextrans are problematic in that (1) their yield is low, (2) the phosphorylation of dextrans with a molecular weight of 100,000 or more is difficult, and (3) only half of the hydroxyl groups at position six can be phosphorylated, and so on (see non-patent documents 2 and 3).

If the biological activities of phosphorylated dextrans can be elucidated, pharmaceutical agents and food products effective for diseases can be developed based on this obtained knowledge.

The database abstract 1976-38836X (Database WPI Week 197621, published 6 April 1976, Thomson Scientific, London, GB) describes a process for manufacturing dextran polyphosphoric esters (DPP), however, does not disclose any experimental data with regard to the alleged biological activity of the DPPs obtained from the manufacturing process described therein.

<Non-patent document 1>

Baba, M., Pauwels, R., Balzarini, J., Arnout, J., Desmyter, J., and De Clercq, E., "Mechanism of inhibitory effect of dextran sulfate and heparin on replication of human immunodeficiency virus in vitro", Proceedings of the National Academy of Science of the United States of America, Vol. 85, p. 6132-6136 (1988);

<Non-patent document 2>

Suzuki, M., Mikami, T., Matsumoto, T. and Suzuki, S., "Preparation and antitumor activity of o-palmitoyldextran phosphates, o-palmitoyldextrans, and dextran phosphate", Carbohydrate Research, Vol. 53, p. 223-229 (1977);

<Non-patent document 3>

Whistler, RL. and Towle, GA., "Preparation and characterization of polysaccharide phosphates", Archives of Biochemistry and Biophysics., Vol.13, p.396-401 (1969)

Disclosure of the Invention

The present invention has been made under such circumstances, and aims to discover the biological activities of phosphorylated dextrans. The present invention also aims to provide pharmaceutical compositions that comprise a phosphorylated dextran as an active ingredient, based on knowledge regarding the discovered biological activities.

In order to resolve the above-described challenges, the present inventors analyzed the biological activities of phosphorylated dextrans. First, the inventors succeeded in chemically introducing phosphate groups into dextrans of various molecular weights, according to the method of Suzuki *et al.* Upon analyzing the biological activities of the produced phosphorylated dextrans, the present inventors discovered that phosphorylated dextrans of any molecular weight have immunopotentiating activity, and specifically, an activity to significantly induce blastogenic activity in mice spleen cells. Such blastogenic activity is not seen in nonphosphorylated dextrans at all. Thus, the present inventors have revealed, for the first time, that immunopotentiating activity can be induced by introducing phosphate groups into dextrans. Although dextran sulfates have been reported as activation factors that polyclonally activate mouse B cells, there is no report that describes phosphorylated dextran derivatives as showing blastogenic activity.

Phosphorylated dextrans have also been revealed to be B cell mitogens, and to activate dendritic cells. Furthermore, phosphorylated dextrans induce IL-10 and IFN-γ, and are therefore expected to have preventive or therapeutic effects on infectious diseases, allergic diseases, or colitis. Thus, compositions comprising a phosphorylated dextran as an active ingredient are expected to be applicable as pharmaceutical agents for preventing or treating infectious diseases, allergic diseases, and colitis.

Interest in food and health is increasing, and it would be highly advantageous from the viewpoint of preventive medicine if the benefits of biological activities, such as the immunopotentiating activities of phosphorylated dextrans, could be obtained from daily food. Compositions comprising phosphorylated dextrans are quite promising as foods for preventing infectious diseases, allergic diseases, or colitis.

The present invention relates to pharmaceutical compositions with immunopotentiating activity, comprising a phosphorylated dextran. More specifically, the present invention provides a phosphorylated dextran as active ingredient for use in preventing or treating infectious diseases, colitis, or allergic diseases. The invention is defined by the claims.

The present inventors analyzed the functions of phosphorylated dextrans, and as a result, discovered that they have novel biological activities. Specifically, the inventors revealed, for the first time, that immunopotentiating activities could be induced by introducing phosphate groups into dextrans. Thus, the present invention provides agents with immunopotentiating activity, that comprise a phosphorylated dextran as an active ingredient.

Dextrans (α-1,6-glucan) are viscous glucans, which mainly comprise α-1, 6 linkages, and are produced from sucrose by *Leuconostoc mesenteroides* and such, which belong to lactic acid bacteria. Typically, dextran is synthesized by transferring a glucose residue from a sucrose molecule to the primer via an α-1,6-linkage, by the action of dextran sucrase. To date, several dozen types of dextran-producing bacteria have been found. Though the α-1,6 linkage content varies according to the bacterial strain, glucans comprising 65% or more α-1,6 linkage content are generally called "dextrans". α-1,3 and α-1,2 linkages are also comprised as other linkages, but most are present as branches.

Dextrans to be used in the present invention are available in the market, and can thus be easily obtained. Dextrans can also be prepared from microorganisms or such using conventional methods used by those skilled in the art.

In the phosphorylated dextrans of the present invention, a phosphorylation site would typically be the hydroxyl group at position six in a dextran. However, in general, it is difficult to accurately regulate which sites in a dextran to phosphorylate. Therefore, the phosphorylated dextrans of the present invention are not limited to those whose position six hydroxyl group is phosphorylated. Generally, it is also difficult to accurately control the ratio of phosphate group introduction (phosphorylation ratio). Thus, there is no limitation as to the ratio of phosphate group introduction for the phosphorylated dextrans of the present invention.

The term "immunopotentiation" refers to activating or enhancing the depressed immune response activities of a host. Those skilled in the art can assay immunopotentiating activities by typically using blastogenic or mitogenic activity on lymphocytes as an indicator. More specifically, immunopotentiating activities can be assayed by measuring blastogenic activity on T cells or B cells. The term "blastogenesis" typically refers to a phenomenon in which lymphocytes stimulated with antigens or mitogens undergo morphological changes via blast formation, change into cells with blast cell characteristics, and become more functional lymphocytes.

The present inventors analyzed the activity of phosphorylated dextran in inducing CD69 expression in mouse spleen cells, and revealed that phosphorylated dextrans enhance CD69 expression in a CD45R-positive cell population, and that this enhancing activity is greater than that of dextrans. CD69 is one of the cell surface antigens expressed in the early stages after lymphocyte activation. When lymphocytes are activated, adhesion molecules are expressed on cell surfaces. Specific adhesion between cells or to the substances surrounding cells via these adhesion molecules plays an important role in the mechanisms of cellular differentiation, proliferation, and functional regulation. CD45R is a cell surface antigen expressed on the cell surface of B cell precursors and mature B cells. This result therefore suggests that phosphorylated dextrans have blastogenic activity and are B cell mitogens. Thus, the phosphorylated dextrans of the present invention have immunopotentiating activity; more specifically, they have mitogenic or blastogenic activity.

The present inventors also used RT-PCR methods to analyze cytokine gene expression in response to stimulation by phosphorylated dextrans in mouse spleen cells, and revealed that phosphorylated dextrans induce IL-10 (a Th2 type cytokine) and IFN-$\gamma$ (a Th1 type cytokine) in mouse spleen cells. Thus, the activity of inducing interferon $\gamma$ (IFN-$\gamma$) or interleukin 10 (IL-10) is an example of a specific immunopotentiating activity of the phosphorylated dextrans of the present invention.

The present invention provides pharmaceutical compositions for use in preventing or treating infectious diseases, colitis, or allergic diseases, where the compositions comprise a phosphorylated dextran as an active ingredient. The present inventors analyzed the physiological functions of phosphorylated dextrans, and discovered that phosphorylated dextrans significantly induce blastogenic activity on mouse spleen cells. Phosphorylated dextrans were also revealed to be B cell mitogens and to activate dendritic cells. Furthermore, since phosphorylated dextrans induced IL-10 and IFN-$\gamma$, the above-described pharmaceutical compositions of the present invention are expected to have preventive or therapeutic effects on infectious diseases, colitis, and allergic diseases.

The term "prevention" in the present invention includes not only prevention of disease onset, but also prevention of disease recurrence after treatment. Specifically, the "allergic diseases" that are a target for prevention or treatment according to the present invention include allergic rhinitis, allergic conjunctivitis, bronchial asthma, atopic dermatitis, intestinal allergies, anaphylactic shock, and such, caused by pollen, mites, house dust, or the like, but are not limited to these diseases.

The pharmaceutical compositions for prevention or treatment of infectious diseases, colitis, and allergic diseases of the present invention are prepared as generic medical formulations when being used. For example, the medicaments of the present invention are prescribed in forms suitable for oral or parenteral administration, as a formulation such as a pharmaceutical composition or a tablet, pill, powder, granule, encapsulated formulation, troche, syrup, solution, emulsion, suspension, or injection; obtained by mixing with a carrier acceptable for drug formulation (excipient, binder, disintegrant, corrigent for taste, corrigent for smell, emulsifier, diluent, solubilizing agent, etc.).

Examples of excipients include lactose, cornstarch, white sugar, glucose, sorbitol, plasma cellulose, and such. Examples of binders include polyvinyl gum arabia, tragacanth, gelatin, shellac, hydroxypropyl cellulose, hydroxypropyl starch, polyvinylpyrrolidone, and such.

Examples of disintegrants include starch, agar, gelatin powder, crystalline cellulose, calcium carbonate, sodium bicarbonate, calcium citrate, dextran, pectin, and such. Examples of lubricants are magnesium stearate, talc, polyethylene glycol, silica, hardened vegetable oil, and such. Further, coloring agents acceptable for addition into medicaments may be used. Examples of corrigents for taste and smell include cocoa powder, menthol, aromatic acids, peppermint oil, Borneo camphor, cinnamon powder, and such. These tablets and granules may be appropriately coated as necessary with sugar coating, gelatin coating, and so on.

In injection preparation, a pH regulator, buffer, stabilizer, preservative, and such, are added as necessary to make subcutaneous, intramuscular, or intravenous injections by conventional methods. Injections can be formulations that are prepared just before use by storing the solution in a container, then producing a solid formulation by freeze-drying and such. A single dose can be stored in a container, and doses can be stored in the same container.

A dosage of a pharmaceutical composition for prevention or treatment of infectious diseases, colitis, and allergic diseases of the present invention is determined by considering the type of dosage form, administration method, age and weight of the subject (mammals including humans), condition of the subject, and so on. Specifically, for an adult patient, for example, 0.01 to 600 mg a day can be administered orally as one to several doses. Examples of doses are more preferably 0.1 to 400 mg/day, and even more preferably 1 to 200 mg/day. Although these doses vary depending on the weight and age of the patient, and on the administration method, one skilled in the art can appropriately select a correct dose. The administration period is also preferably determined so as to be appropriate to the healing course of the patient, and such.

Also described herein are food compositions for preventing or improving infectious diseases, colitis, or allergic diseases, comprising a phosphorylated dextran as an active ingredient. The food compositions include, for example, health foods, functional foods, specified health foods, nutritional supplements, enteral nutritions, and such, but are not limited to these foods as long as they have the effect of preventing or improving infectious diseases, colitis, or allergic diseases. Methods for producing such compositions are well known and frequently used techniques for those skilled in the art. Specifically, the phosphorylated dextrans can be processed into health foods, functional foods, specified health foods, nutritional supplements, enteral nutritions, and such by mixing them with compositions that are acceptable in terms of food hygiene. For example, compositions such as stabilizers, preservatives, coloring agents, fragrances, and vitamins can be appropriately added to and mixed with the above-described phosphorylated dextrans, and then prepared by a conventional method into a form suitable for compositions, such as a tablet, particulate, granule, powder, capsule, liquid, cream, or beverage.

Also described herein are methods for immunopotentiating cells, which comprise the step of contacting cells with a phosphorylated dextran. In the above-described methods, phosphorylated dextrans preferably added to cells to be immunopotentiated, and these cells are incubated. Cells to which the methods described above can be applied include cells that make up tissues associated with immunity, for example, lymphocytes, macrophages, dendritic cells, and such, which are present in the spleen, the Peyer's patch in the intestinal tract, or in nasal mucosal tissue, but are not limited to these cells. Spleen cells or dendritic cells to be used in the present invention are not limited to cells derived from mice, and human-derived cells can also be used.

More specifically, the methods described above can be practiced according to procedures described below in the Examples, but are not limited to such procedures. Those skilled in the art could appropriately modify and carry out the methods described below in the Examples. The term "immunopotentiation" in the methods described above can specifically refer to "blastogenic activity" or to "induction of interferon $\gamma$ or interleukin 10".

Also described herein are methods for producing phosphorylated dextrans, which comprise a step of reacting dextrans with polyphosphoric acid in formaldehyde. The above-described methods for producing phosphorylated dextrans of the present invention are methods that partially modify the conventional methods previously described. Suzuki et *al.* synthesized a phosphorylated dextran by introducing phosphate groups into a dextran (molecular weight of 38,000) by reaction with polyphosphoric acid in formamide solution. The advantages of the methods of the present invention over the conventional methods of Suzuki et *al.* include (1) the yield is increased by 30% compared with conventional methods; (2) dextrans with a molecular weight of 100,000 or more, which conventional methods could not phosphorylate, can be phosphorylated; and (3) almost all hydroxyl groups at position six can be phosphorylated by the present methods, while only half were phosphorylated by the previous methods.

Specifically, the above-described methods can be carried out as follows:

(1) For more efficient reaction, dextran is sufficiently dried, dehydrated formamide is added, and the resulting mixture is stirred (for one hour) in a water bath at 70°C, to completely dissolve the dextran.
(2) Dehydrated triethylamine and polyphosphoric acid are added to the mixture, which is stirred again at 70°C until polyphosphoric acid completely dissolves (two hours).
(3) After that, the solution is left undisturbed for 24 hours to allow phosphorylation to occur.
(4) Phosphorylated dextran is recovered by precipitating with cold ethanol.

In the above-described methods, dextrans and polyphosphoric acid are preferably reacted while heated. The previous method of Suzuki *et al.* does not comprise this heating step. It is thus presumed that those components of the starting dextrans with an especially high molecular weight could not be phosphorylated because this method lacks the heating step. By adding this heating step, even dextran with a molecular weight of 2,000,000 can be phosphorylated. Furthermore, instead of the methanol used in the method of Suzuki *et al.,* ethanol, which is safer, can be suitably used in the methods disclosed herein. This modification enables application to pharmaceutical agents and foods.

More specifically, the above-described methods for producing the phosphorylated dextrans can be carried out by the procedures described below in the Examples, but are not limited to these procedures.

For example, the phosphorylated dextrans can be prepared by a method comprising the following steps of:

(a) reacting a dextran with a phosphate buffer under heat;
(b) freeze-drying the reaction solution of step (a); and
(c) heating the freeze-dried sample of step (b) at 100-160°C for 24 hours.

The heating conditions for the freeze-dried sample in step (c) of the above method are preferably 140-160°C for 24 hours, and more preferably 160°C for 24 hours.

An example of an above method is: suspending 300 mg of a polysaccharide sample (a dextran) in 10 ml of 0.1 M phosphate buffer (pH5.5), heating the mixture to 70°C while stirring, and then stirring until the dextran is completely dissolved. Then, freeze-drying the sample solution, and heating at 140°C to 160°C for 24 hours.

Since the above-mentioned methods utilize a phosphorylation method using safe reagents, the compositions comprising a phosphorylated dextran of the present invention are deemed to be safe as foods or pharmaceutical agents. Those skilled in the art can appropriately modify and carry out the methods (procedures) described below in the Examples.

Brief Description of the Drawings

Fig. 1 is a graph showing the results of anion exchange chromatography of the phosphorylated dextrans at each phosphorylation reaction time. Phosphorylated dextrans were prepared by the heated reaction of dextrans with polyphosphoric acid in a formaldehyde solution.

Fig. 2 is a graph showing changes in the phosphorus content of phosphorylated dextrans at each phosphorylation reaction time.

Fig. 3 is a graph showing the blastogenic activity of phosphorylated dextrans on mouse spleen cells. White bars indicate dextrans. Black bars indicate phosphorylated dextrans. Lipopolysaccharide, 89.3***; concanavalin A, 189.4*** (significant differences against the non-stimulated control: *P<0.05, **P<0.01, ***P<0.001).

Fig. 4 is a graph showing changes in the blastogenic activity of phosphorylated dextrans with stimulation concentration. Lipopolysaccharide, 79.8***; concanavalin A, 247.8*** (significant differences against the non-stimulated control: *P<0.05, **P<0.01, ***P<0.001).

Fig. 5 is a graph showing changes in the blastogenic activity of phosphorylated dextrans over time. *P<0.05, **P<0.01 (significant differences against the non-stimulated control).

Fig. 6 is a graph showing the blastogenic activities of phosphorylated dextrans on T cells (left) and B cells (right).

Fig. 7 shows the results of flow cytometry, showing enhanced CD86 expression by phosphorylated dextrans in dendritic cells.

Fig. 8 is a continuation of Figure 7.

Fig. 9 is a photograph showing the induction of cytokine gene expression in mouse spleen cells by phosphorylated dextrans. Lane 1 indicates a control (water) ; lane 2, a dextran; and lane 3, a phosphorylated dextran.

Fig. 10 is a graph showing the results of measurement of dextran phosphorylation at each temperature by using anion exchange chromatography. After reacting dextrans with phosphate buffer under heat, and freeze-drying the reaction solutions, the freeze-dried samples were heated at 100-160°c for 24 hours to produce phosphorylated dextrans.

Fig. 11 is a graph showing changes in the phosphorus content of phosphorylated dextrans at each temperature. After reacting dextrans with phosphate buffer under heat, and then freeze-drying the reaction solutions, the freeze-dried samples were heated at 100-160°c for 24 hours, producing phosphorylated dextrans.

Fig. 12 is a graph showing changes in the blastogenic activity of phosphorylated dextrans at each phosphorylation reaction temperature. After reacting dextrans with phosphate buffer under heat, and freeze-drying the reaction solutions, the freeze-dried samples were heated at 100-160°c for 24 hours, producing phosphorylated dextrans. (*P<0.05, **P<0.01).

## Best Mode for Carrying Out the Invention

Herein below, the present invention will be specifically illustrated with reference to examples, but is not to be construed as being limited thereto.

DEXTRAN 40,000 Da (Wako Pure Chemical Industries) and DEXTRAN with average molecular weights of about 10,500, 160,000, 513,000, and 2,000,000 Da (SIGMA CHEMICAL CO., St. Louis, MO, USA) were used in the experiments.

Significant differences between the experimental groups and the control groups were evaluated using Student's t-tests.

[Example 1] Preparation of phosphorylated dextrans

Dextran phosphorylation was carried out using the partially modified methods of Whistler *et al.* and Suzuki *et al.* Specifically, a dextran (100 mg; dextrans dried in the presence of $P_2O_5$ under reduced pressure for 24 hours were used) was suspended in dehydrated formamide (10 ml; the supernatant resulting from adding a molecular sieve (4A 1/16; Wako Pure Chemical Industries) to a commercially available special grade reagent and standing it for 24 hours was used). This suspension was stirred in a 70°C water bath for one hour until the dextran was completely dissolved. Then, dehydrated triethylamine (2 ml of the supernatant resulting from adding KOH pellets to a commercially available special grade reagent and standing it for 24 hours was used) and polyphosphoric acid (500 mg) were added. Likewise, the resulting mixture was stirred in a 70°C water bath until polyphosphoric acid was completely dissolved (for two hours or longer). The reaction solution was then allowed to stand at room temperature for 24 hours to phosphorylate the dextran. After the reaction was complete, two volumes of cold ethanol were added to the solution, and the resulting precipitate (ethanol precipitation) was collected by centrifugation. This precipitate was dissolved in Milli Q water (100 ml), the pH was adjusted to 9.0 using 10% NaOH solution, and this was then desalted by two days of dialysis against Milli Q water. The dialysate was concentrated using a rotary evaporator, and then freeze-dried to give the "crude phosphorylated dextran".

The crude phosphorylated dextran was then dissolved at a concentration of 20 mg/ml in 50 mM Tris-HCl buffer (pH 8.6), and applied to an anion exchange chromatography using HiTrapQ HP (1.6 x 2.5 cm; Amersham Pharmacia Biotech UK, Buckinghamshire, England). The column was washed with five times the column's volume of 50 mM Tris-HCl buffer (pH 8.6), and then gradient-eluted from 0 M to 1.0 M NaCl. Neutral sugars in the eluted fractions were monitored by the phenol-sulfuric acid method, and the adsorbed materials were recovered based on this elution curve. The fractions were desalted by two days of dialysis against Milli Q water. The dialysate was concentrated using a rotary evaporator, and then freeze-dried to give the "purified phosphorylated dextran". The conditions used in the anion exchange chromatography were:

Anion exchange chromatography:

Column: HiTrap Q HP (1.6 x 2.5 cm)
Mobile phase: 50 mM Tris-HCl buffer (pH 8.6)
Elution: a linear gradient elution using the above buffer containing 0-1.0 M NaCl
Flow rate: 5.0 ml/min
Detection: phenol-sulfuric acid method (at 490 nm; to detect neutral sugars)

Nearly 100% of the dextrans of each molecular weight (molecular weights of about 10,000, 40,000, 160,000, 510,000, and 2,000,000) were confirmed using anion exchange chromatography to be phosphorylated.

[Example 2] Dextran phosphorylation in various time periods

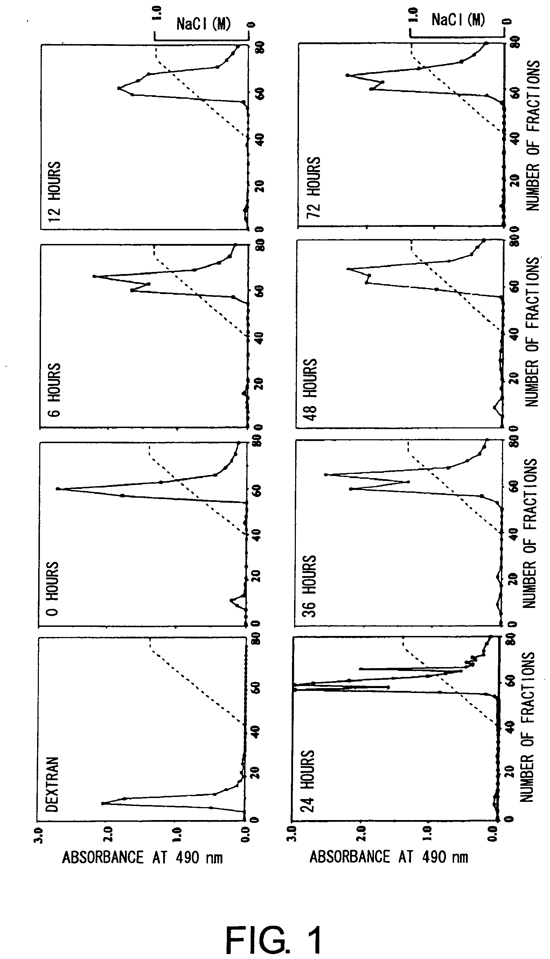

To determine the optimal conditions for dextran phosphorylation, the phosphorylation of a dextran (molecular weight of 2,000,000) was compared over time to investigate optimal reaction time. Phosphorylation reactions were carried out at 0, 6, 12, 24, 36, 48, and 72 hours. When unphosphorylated dextran was applied to anion exchange chromatography, it was all eluted in the flow-through fraction. The time when the polyphosphoric acid had completely dissolved in the phosphorylation reaction solution was taken as a reaction time of zero hours, and phosphorylations were carried out for various time periods within 72 hours.
As a result, even at a reaction time of zero hours, about 90% of the total dextran was adsorbed to the anion exchange column. Furthermore, about 100% of dextran was adsorbed to the column at 6, 12, 24, 36, 48, and 72 hours of reaction (Fig. 1). However, since similar amounts of dextran were adsorbed over 6 to 72 hours, an optimal reaction time could not be determined based on the anion exchange chromatography.

[Example 3] The phosphorus content in phosphorylated dextrans

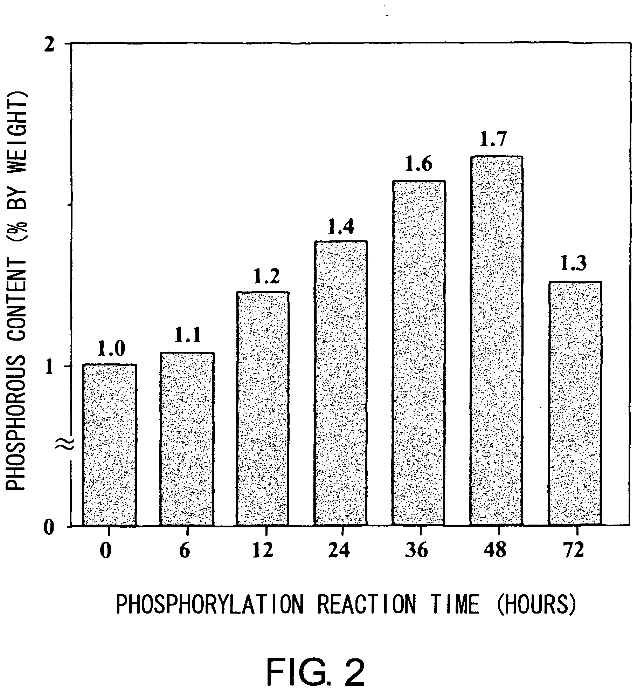

The efficiency of dextran phosphorylation at each reaction time was estimated by measuring the phosphorus contents of the phosphorylated dextrans, and the optimal reaction time was thus determined.
The mass of phosphorus contained in a phosphorylated dextran was determined by the method of Dittmer *et al.* Specifically, 1 ml of phosphorylated dextran solution (1 mg/ml) was transferred into a test tube, and dried at 60°C while spraying $N_2$ gas. 0.4 ml of 70% perchloric acid was added to the sample, and the sample was then heated for about 20 minutes until becoming clear. After decomposition, the sample was cooled to room temperature. Then, 2.4 ml of ammonium molybdate reagent (4.4 g of special grade ammonium molybdate is dissolved into 200-300 ml of distilled water, 14 ml of analytical grade sulfuric acid is added to this, and the volume is then adjusted to 1000 ml) and 2.4 ml of a reducing reagent (Fiske & Subbarow's reducing reagent; 30 g of anhydrous sodium bisulfite, 6 g of anhydrous sodium sulfite, and 0.5 g of 1,2,4-aminonaphtholsulphonic acid were mixed and grinded in a mortar; the mixture was dissolved into 250 ml of distilled water, allowed to stand in a dark place for three hours, and then filtered and stored in a brown bottle; this solution was diluted to 1:12 and used) were added to the sample and mixed. The mixture was heated at 100°C for ten minutes. After standing to cool, the absorbance of the solution at 830 nm was measured. The mass of phosphorus contained in the sample was calculated from a standard curve prepared using potassium dihydrogen phosphate. The phosphorus content in each phosphorylated dextran is shown in Fig. 2.
In the results, the reaction time of zero hours had the lowest phosphorus content of about 1.0%. After that, the phosphorus content gradually increased with reaction time, reaching a maximum of about 1.7% at 48 hours of reaction. At 72 hours, the phosphorus content had decreased to about 1.3%.
Since the value of phosphorus content in phosphorylated dextrans serves as an indicator for phosphorylation efficiency, the optimal reaction time for phosphorylation was determined to be 48 hours, at which the phosphorus content marked the maximum.

[Example 4] Preparation of mouse spleen cells

In the preparation of immune cells, specific pathogen free (SPF) BALB/c mice (Japan SLC, Shizuoka, Japan) were used for examination. Five-week old male mice were purchased, and allowed to freely consume distilled water and MR breeder (Nihon Nosan Kogyo (Nosan Co.)), which is experimental feed for mice and rats. Six- to ten-week-old mice were used for the experiments.

BALB/c mice were anesthetized with ether, and then sacrificed by exsanguination. Their spleens were excised and washed with phosphate buffer (PBS-4x S.P; ph 7.3) containing streptomycin and penicillin (400 mg and 400 U/ml, respectively) to remove adipose tissue and such. The spleens were well dispersed in gauze, and spleen cells were filtered through the gauze into PBS to prepare a cell suspension. This cell suspension was centrifuged (at 300x g for five minutes at 4°C). After discarding the supernatant, the cells were re-suspended in RPMI-1640 medium (SIGMA). The suspension was filtered through a steel mesh to remove aggregated cells, and centrifuged again (five minutes at 300x g and 4°C). After discarding the supernatant, the cells were finally re-suspended in RPMI-1640 containing 2% fetal calf serum (FCS: Biocell Lab., Inc., CA, US). The viable cell count was determined using a trypan blue staining method, and this suspension was used as the spleen cell suspension in the experiments.

[Example 5] The blastogenic activity of phosphorylated dextran on mouse spleen cells

The cell suspension was transferred into each well of a 96-well microplate (SUMITOMO BAKELITE CO., LTD. Tokyo, Japan) to give $2x 10^5$ cells per well. The dextran and five types of phosphorylated dextrans (molecular weights of about 10,000, 40,000, 160,000, 510,000, and 2, 000, 000) prepared in Example 1 were respectively added to the wells to a concentration of 100 $\mu$g/ml. The cells were then cultured in RPMI-1640 (2% FCS) under 5% $CO_2$ at 37°C for 48 hours. As positive control samples, lipopolysaccharide (LPS: B-cell mitogen, *E.coli* 0111: B4, SIGMA) or concanavalin A (ConA: T-cell mitogen, SIGMA) were added at a final concentration of 20 $\mu$g/ml and 2 $\mu$g/ml, respectively. 16 hours before the end of the incubation, methyl- [$^3$H]-thymidine ([$^3$H]TdR; Amersham Pharmacia Biotech) was added at 9.25 kBq per well for pulse labeling. After incubation was complete, the cells were collected onto glass filters (LABO MASH LM 101- 10, LABO SCIENCE CO. , LTD.) using a cell harvester (LABO MASH LM 101- 655, LABO SCIENCE CO., LTD. Tokyo, Japan), and then dried using a dryer. The glass filters were placed into vials designed for a scintillation counter, and 3 ml of a cocktail for a liquid scintillator (0.1 g of POPOP (1,4-bis-[2-(5-phenyloxazolyl)]benzene; Dojindo Laboratories, Kumamoto, Japan) and 4.0 g of DPO (2,5-Diphenyloxazole; Dojindo Laboratories) dissolved in 1 L of toluene) was added to the vials. The amount of [$^3$H] TdR incorporated into the lymphocytes was determined using a liquid scintillation counter (LS1801; Beckman Coulter, Tokyo).

The blastogenic activities on lymphocytes were evaluated by calculating Stimulation Indexes (S.I.) from the [$^3$H] TdR uptake of the cells, using the equation described below:

$$\texttt{S.I.=[(counts per minute in treated) - (counts per minute in background)]/[(counts per minute in control) - (counts per minute in background)]}$$

Furthermore, at the same time, the S.I. values for LPS and ConA used as controls were examined to evaluate cell reactivity and the adequacy of the blastogenesis test.

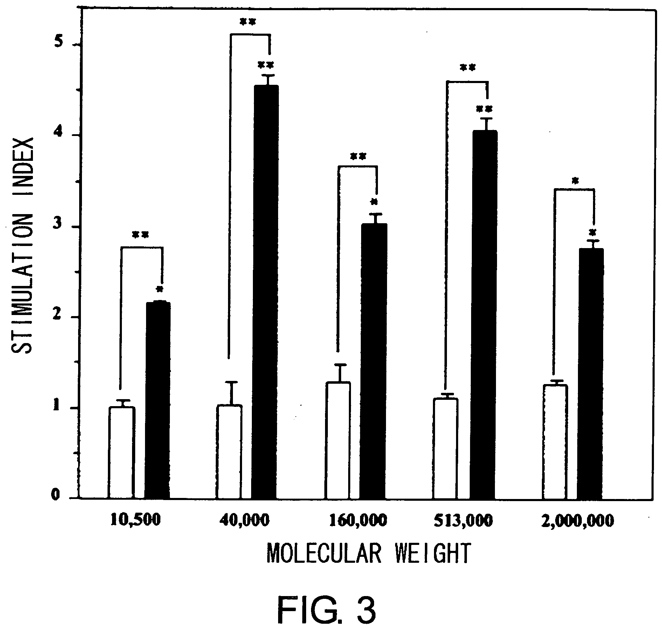

The results indicate that the introduction of phosphate groups significantly induces blastogenic activity on mouse spleen cells for dextrans of every molecular weight (Fig. 3). Of these, the phosphorylated dextran with a molecular weight of 40,000 exhibited the highest S.I. value of 4.5.

[Example 6] Characteristics of lymphocyte blastogenic activities of phosphorylated dextrans

(1) Evaluation of stimulant concentrations in blastogenic activities

The blastogenic activities of phosphorylated dextrans on mouse spleen cells were measured as described in Example 5, except that phosphorylated dextrans were added at concentrations of 100 ng/ml, 1 $\mu$g/ml, 10 $\mu$g/ml, 100 $\mu$g/ml, or 200 $\mu$g/ml, the cells were incubated in RPMI-1640 medium (2% FCS) under 5% $CO_2$ at 37°C for 48 hours, and methyl-[$^3$H]-thymidine uptakes were measured. Blastogenic activity was estimated by calculating S.I. values from the radioactivity, compared with a control.

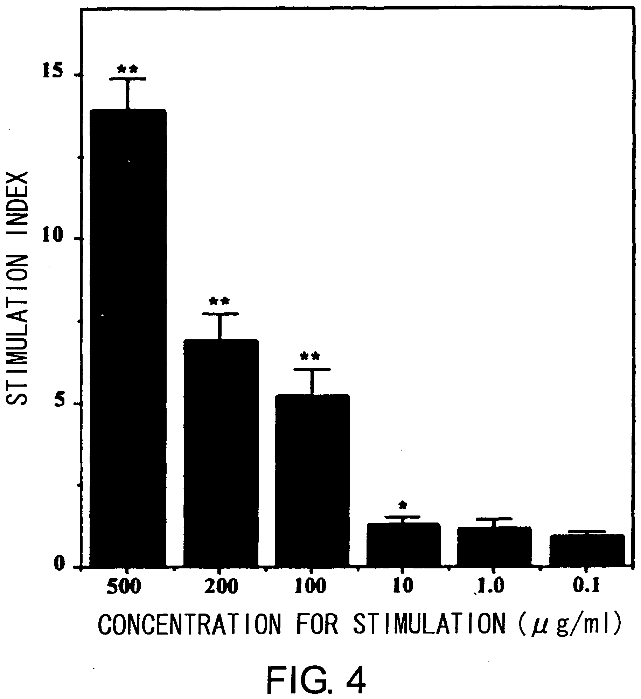

Phosphorylated dextrans significantly induced blastogenic activity at concentrations from 10 to 500 $\mu$g/ml, and showed the highest activity of S.I.=13.9 at 500 $\mu$g/ml (Fig. 4).

(2) Evaluation of incubation time with regards to blastogenic activity

The blastogenic activities of phosphorylated dextrans on mouse spleen cells were determined as described in Example 5, except that the cells were incubated at phosphorylated dextran concentrations of 200 µg/ml for 12, 24, 48, 72, or 120 hours, and methyl- [$^3$H]-thymidine for pulse labeling was added at 9.25 kBq per well, 16 hours before the end of incubation. After the incubation, methyl- [$^3$H]-thymidine uptake was measured. The blastogenic activity was estimated by calculating the S.I. value from the radioactivity, compared with a control.

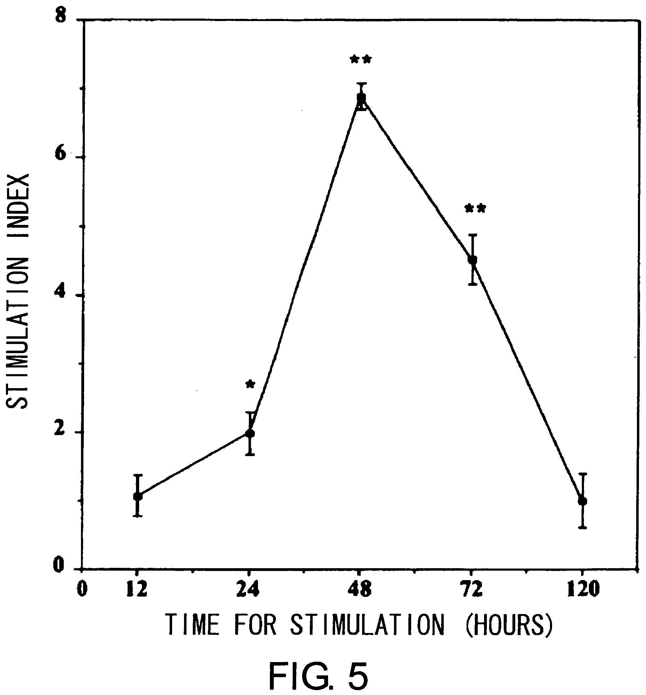

A significant blastogenic effect was induced from an incubation time of 24 hours (Fig. 5), and the highest activity of S.I. = 6.9 was observed at 48 hours. Activity then declined, and at 120 hours was comparable to that in the control.

(3) The blastogenic activities of phosphorylated dextrans on T cells and B cells

The mouse spleen cell suspension prepared in Example 4 was washed by centrifugation (at 2,500 rpm for five minutes at 4°C) with a buffer for cell separation (0.5% BSA, 2mM EDTA/-PBS). 3x 10$^7$ cells were incubated at 4°C for 15 minutes with 50 µl of a 20-times diluted biotinylated anti-mouse CD45R antibody (CLTAG Lab, Burlingame, CA). The cells were washed by centrifugation with the cell separation buffer (five minutes at 2,500 rpm and 4°C), and the supernatant was then discarded. 30 µl of ten-fold diluted streptavidin microbeads (Miltenyi Biotec GmbH, Germany) was added to the cells, and the mixture was incubated at 4°C for 15 minutes. The cells were washed by centrifugation with the cell separation buffer (five minutes at 2,500 rpm and 4°C), re-suspended in the same buffer, and the suspension was then filtered through a nylon mesh to remove aggregated cells. The cell suspension was applied to a magnetic cell sorting system (MACS: Magnetic Cell Sorting System, Miltenyi Biotec GmbH), and was twice passed through a positive selection column (MS$^+$/RS$^+$, Miltenyi Biotec GmbH). The adsorbed fraction was taken as B cells, and the flow-through fraction as T cells. On determining the proportion of each cell type using FACS, the proportion of B cells increased to about 90% after separation, but comprised 19.3% prior to separation, and thus efficient cell separation was achieved. The proportion of T cells after separation was about 97%.

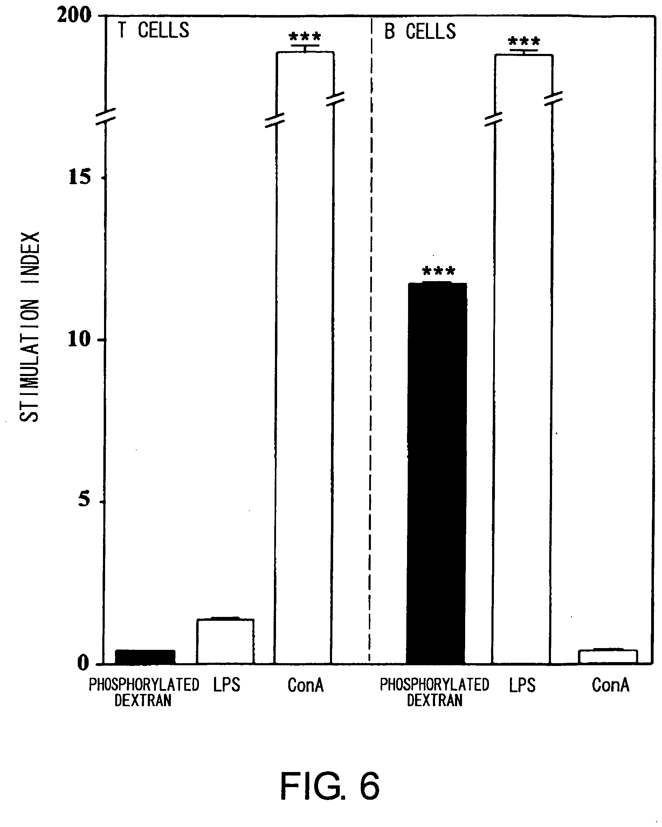

The cells thus obtained were collected by centrifugation and re-suspended in RPMI-1640 containing 10% FCS. The blastogenic activity was then determined using the method described above, except that the stimulation by phosphorylated NPS was carried out at a concentration of 100 µg/ml, and the cells were incubated for 48 hours. As a result, ConA, which is a T cell mitogen, and LPS, which is a B cell mitogen, specifically activated T and B cells respectively, proving the validity of this test using cell fractions. On the other hand, the blastogenic activity of phosphorylated dextrans (100 µg/ml) was observed only for B cells, and the S.I. value was 13 (Fig. 6). This indicates that phosphorylated dextrans are B cell mitogens.

[Example 7] Induction of CD86 expression in mouse spleen cells by phosphorylated dextrans

Mouse spleen cells were transferred onto a 24-well microplate (SUMITOMO BAKELITE CO., LTD. Tokyo, Japan) to give 4x 10$^6$ cells per well in an aliquot of 500 µl. A phosphorylated dextran was added to a concentration of 200 µg/ml. The plate was then incubated under 5% $CO_2$ at 37°C for 24 hours. RPMI-1640 containing 10% FCS was used as a culture medium. After incubation, the cells were collected by centrifugation (five minutes at 2400 rpm and 4°C), and washed with FACS washing buffer (PBS containing 2% FCS and 0.01% $NaN_3$) by centrifugation (five minutes at 2400 rpm and 4°C). The cell surface antigens were determined by immunostaining using anti-CD69 antibody and anti-CD86 antibody. Specifically, 20-fold diluted PE-labeled anti-mouse CD86 antibody (CALTAG), 20-fold diluted FITC-labeled anti-mouse CD8 antibody (Serotec Ltd., Kidlington, UK), and 20-fold diluted biotin-labeled anti-mouse CD11c antibody (CALTAG), were added to the cell pellet in various combinations. The mixtures were mixed well and then allowed to react in the dark for 15 minutes at room temperature. The cells were washed with FACS washing buffer by centrifugation (five minutes at 2400 rpm and 4°C). Then, 10 µl of 100-fold diluted Streptavidin PE-Cy5 (Bioscience, San Diego, CA, USA) was added, and the mixture was allowed to react in the dark for 15 minutes at room temperature. Thus immunostained cells were washed with the FACS washing buffer by centrifugation (five minutes at 2400 rpm and 4°C). After fixing with paraformaldehyde for one hour, the cells were suspended in 1 ml of sheath fluid (Facs Flow, Becton, Dickinson, MA, USA), and the suspension was filtered through a nylon mesh. The amount of bound antibody was analyzed using FACS calibur™ Model 3A (Becton).

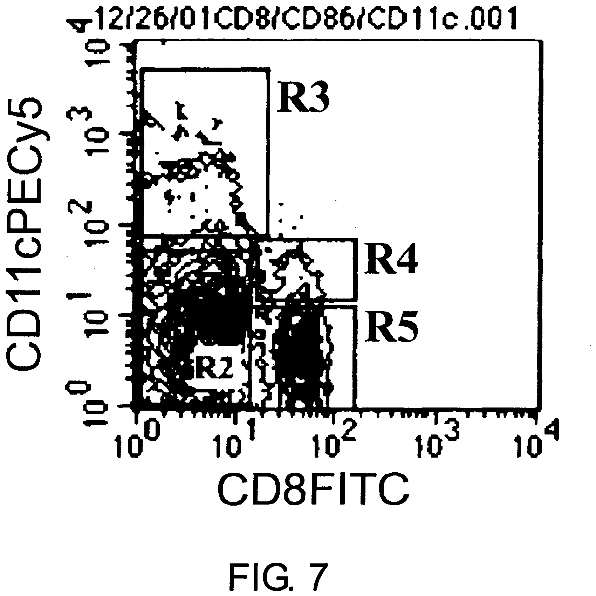

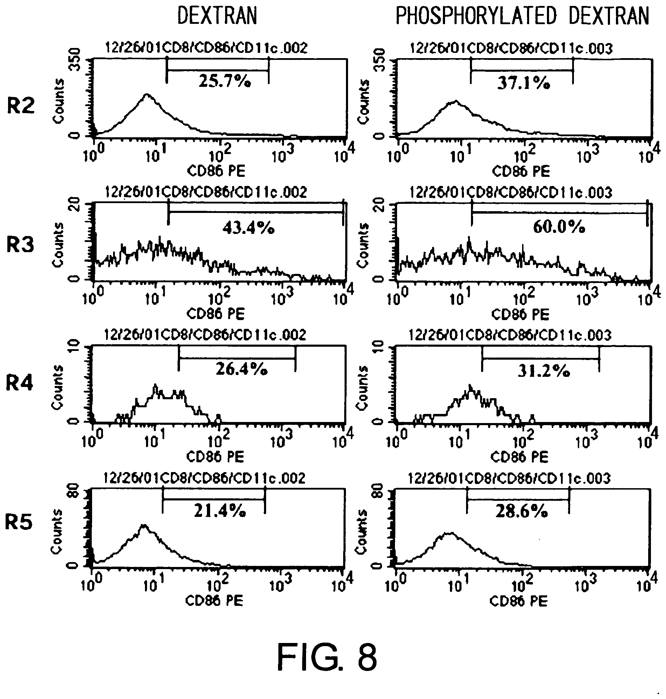

The flow cytometry analysis of the induction of CD86 expression in mouse spleen cells by phosphorylated dextrans (Figs. 7 and 8), showed that phosphorylated dextrans enhance CD86 expression in all cell populations of: CD8$^-$CD11c$^-$ (cells other than CD8$^c$ T cells and DC cells; R2), CD8$^-$CD11c$^+$ dendritic cells (R3 cell population), CD8 $^+$CD11c$^+$ dendritic cells (R4 cell population), and CD8$^+$CD11c$^-$ (CD8$^+$ T cell population; R5). This activity was most prominent for the CD8$^-$CD11c$^+$ dendritic cell population.

[Example 8] Induction of cytokines in mouse spleen cells by phosphorylated dextrans

The expression of cytokine genes in response to phosphorylated dextran stimulation was analyzed by the RT-PCR technique.

The mouse spleen cell suspension was transferred onto a 24-well microplate as a series of 500-$\mu$l aliquots, to give 4x $10^6$ cells per well. A dextran or a phosphorylated dextran was added to a concentration of 200 $\mu$g/ml. The plate was incubated under 5% $CO_2$ at 37°C for 12 hours.

The floating cells were collected into 1.5 ml tubes, and the supernatants were removed by centrifugation (five minutes at 300x g and 4°C). Regarding the adherent cells, cell lysate was recovered by adding 1 ml of TRIzol reagent (GIBCO BRL; Grand island, N.Y) to each well, allowing the plate to stand at room temperature for five minutes, and pipetting the resulting mixture. This cell lysate was then added to the pellet of the floating cells, and the resulting mixture was well pipetted to completely lyse the cells. After allowing the lysate to stand at room temperature for five minutes, 200 ml of chloroform was added, and the mixture was vigorously mixed, and then left undisturbed for three minutes. After centrifugation (15 minutes at 15,000x g and 4°C), the aqueous layer was transferred into new microtubes. 500 ml of isopropyl alcohol was added to the tube and the resulting mixture was well stirred, allowed to stand at room temperature for ten minutes, and centrifuged (15 minutes at 15,000x g and 4°C). The supernatant was then removed. The pellet was washed with cold 75% ethanol (RNase free), the ethanol was allowed to dry, and the pellet was finally dissolved into 30 ml of DEPC-$H_2O$ and used as an RNA sample.

RT-PCR was carried out with RobusT RT-PCR (Daiichi Pure Chemicals, Tokyo). Specifically, RT-PCR was carried out under the conditions shown in Table 1, using the total RNA prepared from mouse spleens as a template, an oligo dT primer (pd(T)12-18), and primers for mouse $\beta$-actin, IL-7, IL-10, IL-12p40, IFN-$\gamma$, and TNF-$\alpha$.

Table 1

Reaction Solution for RT-PCR

| | |
|---|---|
| Roubs T reaction buffer | 1$\mu$L |
| 50mM $MgCl_2$ | 1.5$\mu$L |
| dNTP mix (10 mM each) | 1$\mu$L |
| Template RNA | 500ng |
| Forward primer (10 $\mu$mol) | 1$\mu$L |
| Reverse primer (10 $\mu$mol) | 1$\mu$L |
| AMV reverse transcriptase | 1$\mu$L |
| DNA polymerase | 2$\mu$L |
| DEPC-treated water (add to make the total volume to 50 $\mu$L) | |

RT-PCR reaction

| | | |
|---|---|---|
| 42°C | for 30 minutes | |
| 94°C | for five minutes | |
| 60°C | for five minutes | |
| 94°C | for 15 seconds | |
| 60°C | for 15 seconds | 30 times |
| 72°C | for 30 seconds | |
| 72°C | | |
| 4°C | for ten minutes keep | |

The sequences of the cytokine primers used are shown in Table 2.

Table 2

| Cytokines | Sequences of the primers(5' to 3') |
|---|---|
| $\beta$-actin | forward: TGTGATGGTGGGAATGGGTCAG (SEQ ID NO:1)<br>reverse: TTTGATGTCACGCACGATTTCC (SEQ ID NO:2) |
| IL-7 | forward: GTCACATCATCTGAGTGCCACA (SEQ ID NO:3)<br>reverse: GTAGTCTCTTTAGGAAACATGCATC (SEQ ID NO:4) |

(continued)

| Cytokines | Sequences of the primers(5' to 3') |
|---|---|
| IL-10 | forward: GTGAAGACTTTCTTTCAAACAAAG (SEQ ID NO:5) <br> reverse: CTGCTCCACTGCCTTGCTCTTATT (SEQ ID NO:6) |
| IL-12p40 | forward: CGTGCTCATGGCTGGTGCAAAG (SEQ ID NO:7) <br> reverse: CTTCATCTGCAAGTTCTTGGGC (SEQ ID NO:8) |
| IFN-γ | forward: TACTGCCACGGCACAGTCATTGAA (SEQ ID NO:9) <br> reverse: GCAGCGACTCCTTTTCCGCTTCCT (SEQ ID NO:10) |

The reaction solution obtained by PCR was applied to electrophoresis on a 3% agarose gel, and, after staining with ethidium bromide solution, the result was photographed using FAS-III full system +DS30 (TOYOBO, Tokyo, Japan).

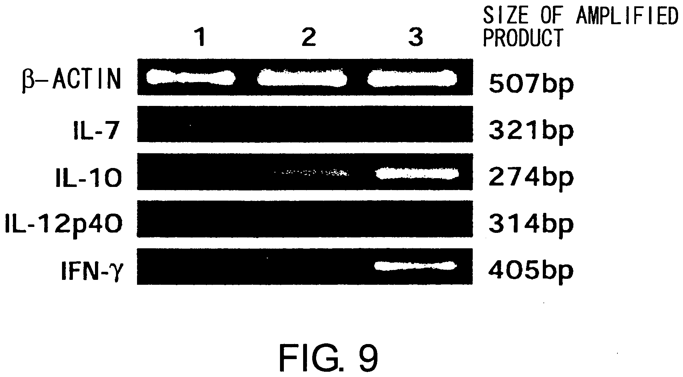

Phosphorylated dextran induced the expression of IL-10 and IFN-γ mRNA in mouse spleen cells (Fig. 9).

Activated dendritic cells are known to produce IFN-γ and induce Th1 immune response. IFN-γ is understood as an important humoral factor in the intercellular network, for regulating not only innate immunity but acquired immunity. On the other hand, IL-10 is known as a cytokine produced by Th2 cells, and in the presence of macrophages is known to suppress cytokine production by Th1 cells. The suppressing effect on IFN-γ production is particularly prominent.

Th1 and Th2 immune responses are inhibitory to each other. A shift in this Th1/Th2 balance toward Th2 is thought to lead to allergic diseases, while a shift toward Th1 is thought to cause inflammatory reactions, such as colitis. Anti-viral activity as well as various immunomodulating functions are expected as a physiological function of IFN-γ. Steidler *et al.* have reported the significant improvement of inflammatory reactions in colitis model mice on the oral administration of *Lactococcus lactis ssp. lactis* expressing IL-10, which has a therapeutic effect on colitis (Steidler, L., Hans, W., Schotte, L., Neirynck, S., Obermeier, F., Falk, W., Fiers, W. and Remaut, E. "Treatment of murine colitis by Lactococcus lactis secreting interleukin-10". Science, 289, 1352-1355 (2000)).

In this way, the individual physiological functions of IFN-γ and IL-10 are diverse, however, with regard to the *in-vivo* cytokine-mediated immunopotentiating activities of phosphorylated dextrans, the expression of that activity can be understood as cytokine interactions, and the effect of phosphorylated dextrans can be assessed based on the interactions.

[Example 9]

(a) Preparation of phosphorylated dextrans

Dextrans were phosphorylated with reference to documented information regarding a phosphate buffer using orthophosphates (Edward Tarelli and Susan F. Wheeler, "Drying from phosphate-buffered solutions can result in the phosphorylation of primary and secondary alcohol groups of saccharides, hydroxylated amino acids, proteins and glycoproteins.", Analytical Biochemistry, 222, 196-201 (1994)). First, 300 mg of a polysaccharide sample was suspended in 10 ml of 0.1 M phosphate buffer (pH 5.5), and the resulting mixture was stirred and heated to 70°C to completely dissolve the polysaccharide sample. Next, the sample solution was freeze-dried, and then heated at 140-160°C for 24 hours.

The sample described above was then dissolved into 30 ml of 10 mM ammonium hydrogencarbonate solution, and dialyzed against 10 mM ammonium hydrogencarbonate solution overnight. The sample was further dialyzed against 50 mM Tris-HCl buffer (pH 8.6) for two days to desalt (to completely remove free phosphate). The sample solution was gradient-eluted using a DEAE-Toyopearl 650M column (1.6 x 11 cm) . The sugar content in the eluate was monitored by the phenol-sulfuric acid method.

(b) Examination of the optimal temperature for dextran phosphorylation

The optimal temperature for dextran phosphorylation was examined using the same method described in the above Examples. Phosphorylation was carried out at 80C, 100, 120, 140, and 160°C. When unphosphorylated dextran was applied in anion exchange chromatography, it was all eluted in the flow-through fraction.

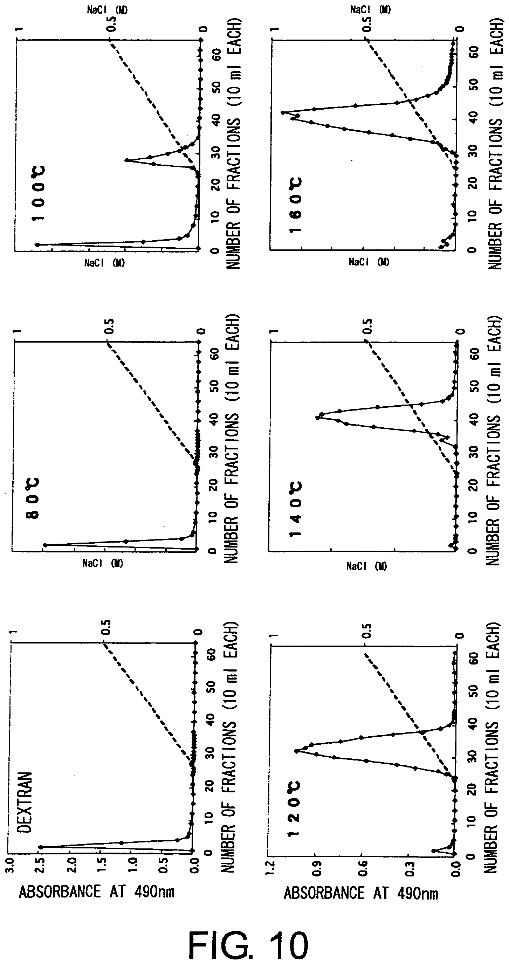

The result showed that, as the phosphorylation temperature increased, the reaction products were more adsorptive to the column, indicating that the degree of phosphorylation became higher (Fig. 10).

Furthermore, the optimal temperature was determined by estimating the dextran phosphorylation efficiencies at each temperature, based on measurements of the phosphorus content in the phosphorylated dextrans, using the same method described above in Example 3.

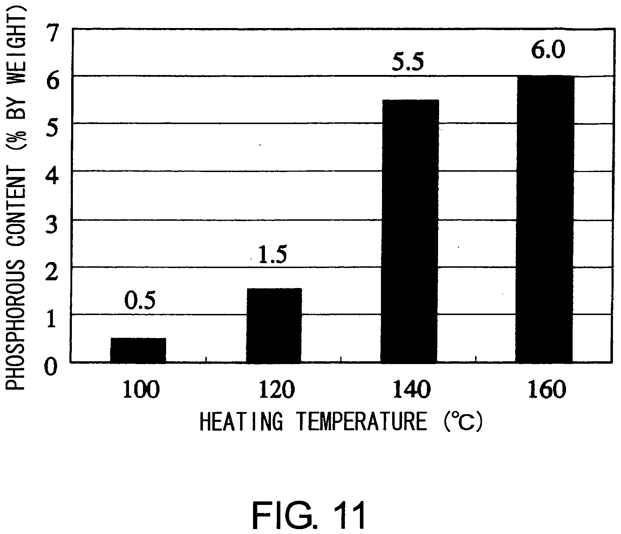

As a result, the phosphorus content showed high values at 140°C and 160°C (Fig. 11). Though some degree of sample browning was observed at 160°C, the optimal heating temperature, where the introduction rate of phosphate group exceeded 5%, was thought to be 140-160°C.

(c) The blastogenic activity of phosphorylated dextrans on lymphocytes

The blastogenic activity on lymphocytes derived from mouse spleens was measured for the phosphorylated dextrans prepared by the method described in the above Example, using the same method described in Example 5. Those phosphorylated dextrans prepared at phosphorylation temperatures of 100, 120, 140, or 160°C were used.

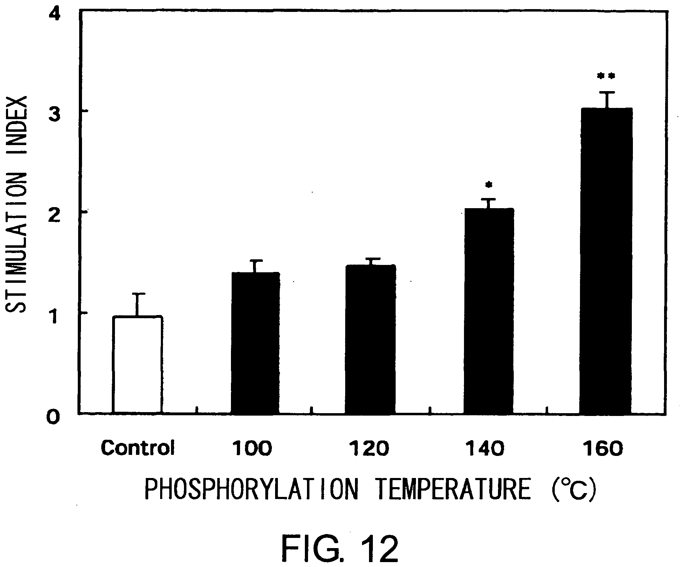

The results confirmed that the phosphorylated dextran prepared at a phosphorylation temperature of 160°C exhibited the highest S.I. value, and thus had the highest lymphocyte blastogenic activity (Fig. 12).

Industrial Applicability

The present invention provides agents comprising a phosphorylated dextran as an active ingredient, and having an immunopotentiating activity. Since phosphorylated dextrans are B cell mitogens and activate dendritic cells, and further comprise the activity of inducing IL-10 and IFN-$\gamma$, the agents of the present invention are expected to be pharmaceutical agents for preventing or treating infectious diseases, allergic diseases, or colitis.

Furthermore, the phosphorylated dextrans of the present invention were revealed to comprise the activity of activating antigen-presenting cells such as dendritic cells, to enhance the antigen-presenting ability of the cells, and to stimulate the acquired immune system. In addition, since the phosphorylated dextrans of the present invention comprise the activity of directly activating B cells, it is thought that the phosphorylated dextrans of the present invention can be used as effective adjuvants, by enhancing the antibody producing ability of B cells.

Furthermore, compositions comprising a phosphorylated dextran are expected as functional foods comprising immunological activities of contributing to the maintenance and improvement of human health.

Since the present invention demonstrates the importance of the phosphate groups in dextran molecules on the expression of immunological activities, the present invention is highly important as fundamental data for the development of functional foods.

## Claims

1. A phosphorylated dextran as active ingredient for use in preventing or treating infectious diseases, colitis, or allergic diseases.

2. Use of a phosphorylated dextran as active ingredient for the preparation of a pharmaceutical composition for preventing or treating infectious diseases, colitis, or allergic diseases.

3. The phosphorylated dextran for the use of claim 1, wherein the prevention or treatment is achieved by the induction of IL-10 or IFN-γ.

4. The use of claim 2, wherein the prevention or treatment is achieved by the induction of IL-10 or IFN-$\gamma$.

5. The phosphorylated dextran for the use of claim 1, which is for oral administration.

6. The use of claim 2, wherein the pharmaceutical composition is for oral administration.

**Patentansprüche**

1. Phosphoryliertes Dextran als Wirkstoff für die Verwendung bei der Vorbeugung oder Behandlung von Infektionskrankheiten, Colitis, oder allergischen Erkrankungen.

2. Verwendung von phosphoryliertem Dextran als Wirkstoff zur Herstellung einer pharmazeutischen Zusammensetzung für die Vorbeugung oder Behandlung von Infektionskrankheiten, Colitis, oder allergischen Erkrankungen.

3. Das phosphorylierte Dextran für die Verwendung gemäß Anspruch 1, wobei die Vorbeugung oder Behandlung durch die Induktion von IL-10 oder IFN-$\gamma$ erreicht wird.

4. Die Verwendung nach Anspruch 2, wobei die Vorbeugung oder Behandlung durch die Induktion von IL-10 oder IFN-$\gamma$ erreicht wird.

5. Das phosphorylierte Dextran für die Verwendung nach Anspruch 1, welches zur oralen Verabreichung vorgesehen ist.

6. Die Verwendung nach Anspruch 2, wobei die pharmazeutische Zusammensetzung zur oralen Verabreichung vorgesehen ist.

**Revendications**

1. Dextrane phosphorylé comme ingrédient actif destiné à l'utilisation dans la prévention ou le traitement des maladies infectieuses, de la colite, ou des maladies d'origine allergique.

2. Utilisation d'un dextrane phosphorylé comme ingrédient actif destiné à la préparation d'une composition pharmaceutique pour la prévention ou le traitement des maladies infectieuses, de la colite, ou des maladies d'origine allergique.

3. Dextrane phosphorylé destiné à l'utilisation selon la revendication 1, dans lequel la prévention ou le traitement est atteint-e par l'induction d'IL-10 ou d'IFN-$\gamma$.

4. Utilisation selon la revendication 2, dans laquelle la prévention ou le traitement est atteint-e par l'induction d'IL-10 ou d'IFN-$\gamma$.

5. Dextrane phosphorylé pour l'utilisation selon la revendication 1, qui est administré par voie orale.

6. Utilisation selon la revendication 2, dans lequel la composition pharmaceutique est administrée par voie orale.

FIG. 1

FIG. 2

FIG. 3

FIG. 4

FIG. 5

FIG. 6

FIG. 7

FIG. 8

EP 1 543 833 B1

FIG. 9

24

FIG. 10

FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002213305 A

- JP 2003050739 A

### Non-patent literature cited in the description

- Database WPI Week 197621. 1976-38836X
- **BABA, M. ; PAUWELS, R. ; BALZARINI, J. ; ARNOUT, J. ; DESMYTER, J. ; DE CLERCQ, E.** Mechanism of inhibitory effect of dextran sulfate and heparin on replication of human immunodeficiency virus in vitro. *Proceedings of the National Academy of Science of the United States of America,* 1988, vol. 85, 6132-6136
- **SUZUKI, M. ; MIKAMI, T. ; MATSUMOTO, T. ; SUZUKI, S.** Preparation and antitumor activity of o-palmitoyldextran phosphates, o-palmitoyldextrans, and dextran phosphate. *Carbohydrate Research,* 1977, vol. 53, 223-229

- **WHISTLER, RL. ; TOWLE, GA.** Preparation and characterization of polysaccharide phosphates. *Archives of Biochemistry and Biophysics.,* 1969, vol. 13, 396-401
- **STEIDLER, L. ; HANS, W. ; SCHOTTE, L. ; NEIRYNCK, S. ; OBERMEIER, F. ; FALK, W. ; FIERS, W. ; REMAUT, E.** Treatment of murine colitis by Lactococcus lactis secreting interleukin-10. *Science,* 2000, vol. 289, 1352-1355
- **EDWARD TARELLI ; SUSAN F. WHEELER.** Drying from phosphate-buffered solutions can result in the phosphorylation of primary and secondary alcohol groups of saccharides, hydroxylated amino acids, proteins and glycoproteins. *Analytical Biochemistry,* 1994, vol. 222, 196-201

SEQUENCE LISTING

<110> MEIJI DAIRIES CORPORATION

<120> Phosphorylated Dextrans

<130> M1-A0201Y1P

<140>
<141>

<150> JP 2002-213305
<151> 2002-07-23

<150> JP 2003-50739
<151> 2003-02-27

<160> 10

<170> PatentIn Ver. 2.1

<210> 1
<211> 22
<212> DNA
<213> Artificial Sequence

<220>

<223> Description of Artificial Sequence:an artificially synthesized primer sequence

<400> 1

tgtgatggtg ggaatgggtc ag          22

<210> 2

<211> 22

<212> DNA

<213> Artificial Sequence

<220>

<223> Description of Artificial Sequence:an artificially synthesized primer sequence

<400> 2

tttgatgtca cgcacgattt cc          22

<210> 3

<211> 22

<212> DNA

<213> Artificial Sequence

<220>

<223> Description of Artificial Sequence:an artificially synthesized primer sequence

<400> 3

gtcacatcat ctgagtgcca ca          22

<210> 4

<211> 25

<212> DNA

<213> Artificial Sequence

<220>

<223> Description of Artificial Sequence:an artificially synthesized primer sequence

<400> 4

gtagtctctt taggaaacat gcatc          25

<210> 5

<211> 24

<212> DNA

<213> Artificial Sequence

<220>

<223> Description of Artificial Sequence:an artificially synthesized primer sequence

<400> 5

gtgaagactt tctttcaaac aaag          24

<210> 6

<211> 24

<212> DNA

<213> Artificial Sequence

<220>

<223> Description of Artificial Sequence:an artificially synthesized primer sequence

<400> 6
ctgctccact gccttgctct tatt        24

<210> 7
<211> 22
<212> DNA
<213> Artificial Sequence

<220>
<223> Description of Artificial Sequence:an artificially synthesized primer sequence

<400> 7
cgtgctcatg gctggtgcaa ag        22

<210> 8
<211> 22
<212> DNA
<213> Artificial Sequence

<220>
<223> Description of Artificial Sequence:an artificially synthesized primer sequence

<400> 8
cttcatctgc aagttcttgg gc        22

<210> 9
<211> 24
<212> DNA
<213> Artificial Sequence

<220>
<223> Description of Artificial Sequence:an artificially synthesized primer sequence

<400> 9
tactgccacg gcacagtcat tgaa        24

<210> 10
<211> 24
<212> DNA
<213> Artificial Sequence

<220>
<223> Description of Artificial Sequence:an artificially synthesized primer sequence

<400> 10
gcagcgactc cttttccgct tcct        24